(19) Europäisches Patentamt
European Patent Office
Office européen des brevets

(11) Veröffentlichungsnummer : **0 013 956**
**B1**

(12) # EUROPÄISCHE PATENTSCHRIFT

(45) Veröffentlichungstag der Patentschrift : **13.10.82**

(21) Anmeldenummer : **80100263.5**

(22) Anmeldetag : **21.01.80**

(51) Int. Cl.³ : **C 07 C 99/00**, C 07 C101/54, C 07 C101/62, C 07 C 79/46, C 07 C 67/08

(54) **Verfahren zur Herstellung von Alkandiol-bis-aminobenzoesäureestern.**

(30) Priorität : **25.01.79 DE 2902740**

(43) Veröffentlichungstag der Anmeldung : **06.08.80 (Patentblatt 80/16)**

(45) Bekanntmachung des Hinweises auf die Patenterteilung : **13.10.82 Patentblatt 82/41**

(84) Benannte Vertragsstaaten : **BE CH DE FR GB IT NL SE**

(56) Entgegenhaltungen :
**DE A 1 720 680**
**DE A 2 419 322**
**DE A 2 511 093**
**US A 3 095 399**

(73) Patentinhaber : **HOECHST AKTIENGESELLSCHAFT**
**Postfach 80 03 20**
**D-6230 Frankfurt/Main 80 (DE)**

(72) Erfinder : **Papenfuhs, Theodor, Dr.**
**Heinrich-Bleicher-Strasse 40**
**D-6000 Frankfurt am Main 50 (DE)**
Erfinder : **Kümmerle, Kurt, Dr.**
**Unter den Eichen 1**
**D-6233 Kelkheim (Taunus) (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 013 956 B1

Imprimerie Jouve, 18, rue St-Denis, 75001 Paris, France

# Verfahren zur Herstellung von Alkandiol-bis-aminobenzoesäureestern

Die Erfindung liegt auf dem Gebiet einer Verfahrensverbesserung zur Herstellung von Alkandiol-bisaminobenzoesäureestern, die als nicht-toxische Härtungsmittel für Urethankunststoffe (s. DE-OS 2 511 093) Verwendung finden können.

Die Bis-aminobenzoesäureester von Alkandiolen werden in Zwei- oder Mehrstufenverfahren hergestellt, wobei man zunächst als Ausgangsverbindungen die Bis-nitrobenzoesäureester der Alkandiole synthetisiert und daraus, vorzugsweise durch katalytische Reduktion, die gewünschten Amino-Verbindungen gewinnt. Zur Herstellung der Nitrobenzoesäureester sind vor allem Herstellungsverfahren bekannt, die in einem Lösemittel durchgeführt werden. Hierbei wird durch Reaktion von 2 Mol einer Nitrobenzoesäure und einem Mol eines Alkandiols unter Abspaltung von 2 Mol Wasser der Bis-ester hergestellt, wobei das entstehende Wasser mit dem Lösemittel ausgeschleppt wird. Allen diesen Lösemittelverfahren ist gemeinsam, daß die Reaktion nicht quantitativ zu Ende läuft und daß selbst unter Verwendung hoher Mengen an Veresterungskatalysator Ausbeuten von über 80 % d. Th. nicht zu erreichen sind. Die besten Ausbeuten von etwa 75 bis 80 % d. Th. werden erst mit Mengen von « Katalysator » von mindestens 15 Mol-%, bezogen auf die eingesetzte Nitrobenzoesäure, erhalten ; es liegt auf der Hand, daß die Anwendung solcher Mengen von « Katalysator » völlig unwirtschaftlich ist. Die mäßigen Ausbeuten bei den Lösemittelverfahren bedingen einen hohen technischen Reinigungs- und Trennaufwand (Filtrationen und Trocknungen aus Lösemittel, Rückgewinnung der nicht umgesetzten Reaktionskomponenten und des Lösemittels) sowie eine hohe Umweltbelastung, teuere Apparaturen und schlechte Raum/Zeit-Ausbeuten.

Es hat nicht an Versuchen gefehlt, die Bis-nitrobenzoesäureester der Alkandiole in quantitativer Umsetzung zu erhalten. Um einen einheitlichen Reaktionsablauf zu gewährleisten, ging man von dem Nitrobenzoylchlorid aus (s. DE-OS 25 11 093), wobei jedoch die Acylierungsreaktion des Alkandiols ebenfalls in einem Lösemittel und unter Verwendung von zumindest stöchiometrischer Mengen von Pyridin durchgeführt werden mußte. Katalytische Pyridinzusätze führen dagegen zu einem erheblichen Anteil an Nebenprodukten. Dieses Verfahren hat somit kein technisches Interesse erlangen können, zumal die Herstellung des Nitrobenzoylchlorids als weiterer Verfahrensschritt die Wirtschaftlichkeit der Gesamtreaktion negativ beeinflußt. — In der belgischen Patentschrift 814 024, welche der deutschen Offenlegungsschift 24 19 322 entspricht, ist ein Verfahren beschrieben, mit dem man versucht hat, die Bis-nitrobenzoesäureester von Alkandiolen ohne Verwendung eines Lösemittels herzustellen, indem man die Umsetzung der Nitrobenzoesäure und des Alkandiols in Substanz ausführte. Die Umsetzung zum Bis-ester verläuft jedoch erst oberhalb einer Temperatur von 220 °C mit technisch interessantem Umsatz, jedoch auch hier nicht einheitlich, weswegen eine Lösemittelreinigung mit anschließender Filtration angeschlossen werden mußte. Darüber hinaus bestehen schwerwiegende Nachteile in der technischen Nutzung : die sehr hohen Reaktionstemperaturen erfordern außer der großen Energiezufuhr aufwendige Apparaturen ; bei den Reaktionsbedingungen sublimiert nicht umgesetzte Nitrobenzoesäure sehr stark, was außer zur unvollständigem Umsetzung zum Zusetzen von Ventilen etc. führen kann.

Thermochemische Untersuchungen zeigen, daß das Reaktionsgemisch oberhalb 200 °C instabil ist und sich in technischen Apparaturen (quasi adiabatische Bedingungen) unkontrolliert und in heftiger exothermer Reaktion zersetzen kann ; die Veresterung muß zudem zweistufig unter Zwischenbildung des Monoesters durchgeführt werden, so daß eine Unterbrechung der Synthese und erneutes Eintragen der Nitrobenzoesäure in das heiße Reaktionsgefäß erforderlich ist.

In der US-Patentschrift 3 095 399 wird hingegen die Veresterung in einem Lösemittel durchgeführt, wobei Diäthylenglykol als Reaktionspartner und Lösemittel in erheblichem Überschuß eingesetzt wird. In diesem erheblich verdünnten Reaktionsmedium kann die Reaktion zwar unter Rückfluß bei einer Temperatur von etwa 180 °C erfolgen, jedoch muß das Veresterungsprodukt zur Weiterverarbeitung erst isoliert werden. Der Reaktionsansatz der Veresterung kann somit nicht direkt in die nachfolgende Hydrierungsreaktion eingesetzt werden, womit die vorteilhafte technische Durchführung der Synthese in einer Eintopfreaktion ausgeschlossen wird.

All diese hier besprochenen Nachteile der bekannten Verfahren zeigen, daß ein erhebliches technisches Interesse bestand, ein verbessertes Verfahren zur Herstellung von Bis-aminobenzoesäureestern von Alkandiolen und deren Nitro-Vorprodukten zu finden. Auch war es erwünscht, die nachfolgende Reduktion der Nitroverbindungen zu den Aminoverbindungen einheitlich durchzuführen und die Aminobenzoesäure-diester aus dem Reaktionsgemisch in reiner Form zu isolieren. Denn solche Reduktionen wurden bisher in technisch äußerst unvorteilhafter Weise mittels Zinn und Mineralsäure (s. BE-PS 814 024 und entsprechende DE-OS 24 19 322) oder mittels Hydrazinhydrat in Gegenwart von Palladium (s. DE-OS 25 11 093) durchgeführt.

Es erschien nun naheliegend, solche katalytischen Reduktionen mit einem Edelmetallkatalysator in mit Wasser nicht mischbaren Lösemitteln, in denen der Nitroester löslich ist, durchzuführen ; aber auch hier ergaben sich technisch erhebliche Aufarbeitungsprobleme durch die Schwerlöslichkeit der polaren Endprodukte im unpolaren Lösemittel und die damit bedingte schwierige und aufwendige Katalysatorabtrennung und -regeneration.

Mit der vorliegenden Erfindung wurde nun ein vereinfachtes und verbessertes Verfahren zur

Herstellung von Bis-(aminobenzoesäure)-alkandiol-diestern durch Veresterung von Alkandiolen, — ausgenommen solchen, bei denen die Hydroxygruppen in 1,4- oder 2,5-Stellung stehen —, mit Nitrobenzoesäuren und anschließende katalytische Reduktion der Bis-(nitrobenzoesäure)-alkandiol-diester-Verbindung in einem polaren, mit Wasser teilweise oder beliebig homogen mischbaren organischen Lösemittel, wie einem niederen aliphatischen Alkohol, gefunden, das dadurch gekennzeichnet ist, daß man die Veresterung in Gegenwart einer aromatischen Sulfonsäure als Katalysator und in Abwesenheit eines Lösemittels und bei einer Temperatur zwischen 150 und 175 °C durchführt.

Ester aus Alkandiolen, bei denen die Hydroxygruppen in 1,4- oder 2,5-Stellung stehen, lassen sich deshalb nicht herstellen, da diese Alkandiole unter den gegebenen Reaktionsbedingungen intramolekular Wasser abspalten und in Tetrahydrofurane übergehen.

Der so erfindungsgemäß hergestellte Bis-(aminobenzoesäure)-alkandiol-diester kann anschließend aus der Lösung des Reaktionsansatzes erfindungsgemäß mittels Wasser abgeschieden und dann in einfacher Weise abgetrennt werden.

Die erfindungsgemäße Durchführung der ersten Verfahrensstufe in Abwesenheit eines Lösemittels unter der Katalyse einer aromatischen Sulfonsäure hat den Vorteil, daß die Reaktionstemperatur der Veresterung überraschenderweise auf etwa 150 bis 170 °C gesenkt werden kann, so daß einfacher ausgelegte Apparaturen einsetzbar sind und kein Sicherheitsrisiko infolge exothermer Zersetzung oberhalb 200 °C besteht. Trotz eines Fest-/Flüssig-Komponentengemisches von 4-5 : 1 besteht eine rührbare Suspension des Reaktionsgemisches und es tritt kein Sublimieren der Nitrobenzoesäure auf. Darüber hinaus kann die Gesamtreaktion vorteilhaft als Eintopfverfahren durchgeführt werden, d.h. ohne Zwischenisolierung des als Zwischenprodukt gebildeten Nitrobenzoesäuremonoesters.

Die Veresterung wird im allgemeinen so durchgeführt, daß man die Reaktionskomponenten (Nitrobenzoesäure, Alkandiol und die aromatische Sulfonsäure als Katalysator) zusammengibt und auf eine Temperatur von bis zu 170 °C erwärmt, wobei das bei der Reaktion entstehende Wasser abdestilliert wird. Sobald 90 % der stöchiometrisch erwarteten Menge an Wasser abdestilliert sind, führt man die Reaktion unter reduziertem Druck zur besseren Abdestillation des Wassers in kurzer Zeit zu Ende. Der erhaltene geschmolzene Nitrobenzoesäurediester kann nun über eine allgemein übliche Kühl- und Konfektioniervorrichtung, z.B. über eine Schuppenwalze oder einen Pastillierapparat, aus dem Reaktionsgefäß ausgetragen werden ; es wird hierbei ein gut handhabbares, nicht staubendes Produkt erhalten, das nachfolgend der katalytischen Reduktion unterworfen werden kann. Bevorzugt und besonders vorteilhaft kann man das erfindungsgemäße Verfahren in folgender Weise, insbesondere als Eintopfverfahren, durchführen : Zu der Mischung des Alkandiols und des Katalysators wird bei einer Temperatur zwischen 60 und 120 °C, vorzugsweise zwischen 100 und 120 °C, die gesamte, zur Herstellung des Diesters erforderliche Menge an Nitrobenzoesäure eingetragen. Die Nitrobenzoesäure und das Alkandiol können vorteilhaft in äquimolaren Mengen eingesetzt werden ; in der Regel setzt man das Alkandiol in leichtem Überschuß ein und verwendet die Nitrobenzoesäure und das Alkandiol vorzugsweise im molaren Verhältnis von 2 : 1,0 bis 1,1. Der Katalysator wird in einer Menge von 0,5 bis 5 Mol-%, vorzugsweise von 1 bis 3 Mol-%, bezogen auf das Alkandiol, eingesetzt. Beim Zusammengeben der Reaktanten bei einer Temperatur bis zu 120 °C, bei welcher noch keine merkliche Veresterung einsetzt, ergibt sich eine rührbare Suspension, eine notwendige Vorbedingung für eine technische Durchführung der Veresterung. Das Reaktionsgemisch wird nach dem Eintragen der Nitrobenzoesäure sodann auf eine Temperatur zwischen 150 und 175 °C, vorzugsweise auf eine Temperatur zwischen 150 und 170 °C, erwärmt und in diesem Temperaturbereich gehalten ; hierbei läuft die Veresterung zügig ab und kann mit Hilfe der Menge des abdestillierten Reaktionswassers problemlos verfolgt werden. Nach 90 %igem Umsatz legt man Vakuum an das Reaktionsgefäß und führt die Reaktion in dem genannten Temperaturbereich in kurzer Zeit unter Abdestillation des Restwassers zu Ende. Der gebildete Diester kann, wie oben angegeben, isoliert werden ; jedoch wird die Reaktion vorteilhaft als Eintopfverfahren weitergeführt, indem man die Reaktionsschmelze des Nitrobenzoesäure-diesters nach Abkühlen auf eine Temperatur von 100 bis 125 °C, vorzugsweise auf 110 bis 120 °C, mit einem polaren, mit Wasser teilweise oder beliebig homogen mischbaren organischen Lösemittel in einer geschlossenen Apparatur verdünnt, zu der entstehenden Suspension des Diesters einen handelsüblichen Hydrierkatalysator, wie Raney-Nickel, Platin oder Palladium, bevorzugt einen Nickelkatalysator auf Siliciumdioxid als Trägermaterial, zusetzt und den Nitrobenzoesäure-diester mittels Wasserstoff, vorzugsweise unter Druck, reduziert.

Solche polaren organischen Lösemittel sind bspw. aliphatische Alkohole, vorzugsweise ein niederes Alkanol, wie beispielsweise Methanol, Äthanol oder Isopropanol, oder bspw. aprotische Lösemittel, wie Dimethylformamid, Dimethylsulfoxid oder Acetonitril.

Man erhält nach der Reduktion eine Lösung des Bis-aminobenzoesäure-alkandiol-diesters, die durch Filtration vom Katalysator befreit wird. Der Ester wird durch Verdünnen der Lösung mit Wasser bis zu einer Konzentration von 10 bis 50 Gew.-%, vorzugsweise von 25 bis 35 Gew.-% an dem polaren Lösemittel ausgefällt ; er ist chromatographisch rein. Die Ausbeute an dem durch Filtration isoliertem und getrocknetem Endprodukt liegt in der Regel über 90 % d. Th bezogen auf die eingesetzte Nitrobenzoesäure, und erreicht nicht selten nahezu theoretische Werte.

Aus der Mutterlauge kann das verwendete polare, mit Wasser teilweise oder beliebig homogen mischbare organische Lösemittel destillativ regeneriert werden.

Die Reduktion im erfindungsgemäßen Verfahren läuft trotz der Unlöslichkeit der Nitrobenzoesäure-

diester-Verbindung in den verwendeten polaren, mit Wasser teilweise oder beliebig homogen mischbaren organischen Lösemitteln und in besonders vorteilhafter Weise ohne die Notwendigkeit, den Veresterungskatalysator abtrennen zu müssen, glatt, leicht und einheitlich, so daß gewünschtenfalls auf hochaktive, teure Edelmetallkatalysatoren verzichtet werden kann und die Verwendung von Nickel auf mineralischen Trägern völlig ausreicht. Der weitere Vorteil dieses Reduktionsverfahrens im erfindungsgemäßen Verfahren liegt neben der Erlangung eines analysenreinen Endproduktes auch in der einfachen Abtrennung des Hydrierkatalysators durch Filtration von dem gelösten Diester und der Abtrennung von Verunreinigungen von dem ausgefällten Diester mit der wäßrig-organischen Mutterlauge, in welcher sie quantitativ gelöst bleiben. Das Verfahren eignet sich insbesondere für Nitrobenzoesäurediester, die die Nitrogruppe in ortho-, in meta- oder insbesondere bevorzugt in para-Stellung gebunden enthalten und die weiterhin in jedem Benzolkern durch Substituenten, vorzugsweise durch einen oder zwei, insbesondere einen Substituenten, aus der Gruppe Halogen, wie insbesondere Chlor, Alkyl, wie insbesondere niederes Alkyl, beispielsweise Methyl, und Alkoxy, wie insbesondere niederes Alkoxy, wie beispielsweise Methoxy oder Äthoxy, substituiert sein können, und deren Alkylenesterrest vorzugsweise ein Alkylenrest von 2 bis 8, insbesondere von 3 bis 5 Kohlenstoffatomen ist, vorzugsweise ein geradkettiger Alkylenrest von 2 bis 8, insbesondere von 3 oder 5 C-Atomen ist.

Geeignete Ausgangsverbindungen für das erfindungsgemäße Verfahren sind demgemäß die o-, m- oder die p-Nitrobenzoesäure sowie deren durch Substituenten, vorzugsweise durch 1 oder 2, insbesondere einen Substituenten, aus der Gruppe Halogen, wie insbesondere Chlor, Alkyl, insbesondere niederes Alkyl, wie beispielsweise Methyl, und Alkoxy, insbesondere niederes Alkoxy, wie beispielsweise Methoxy oder Äthoxy, substituierten Derivate und Alkandiole mit vorzugsweise 2 bis 8 C-Atomen, insbesondere mit 3 bis 5 C-Atomen, insbesondere vorzugsweise geradkettige Alkandiole von 2 bis 8 C-Atomen, insbesondere von 3 oder 5 C-Atomen, wobei Alkandiole, die die beiden Hydroxygruppen in 1,4- oder 2,5-Stellung gebunden enthalten, ausgeschlossen sind.

Die vorliegende Erfindung betrifft somit vorzugsweise die Herstellung von Bis-aminobenzoesäure-alkandiol-diestern der allgemeinen Formel (1)

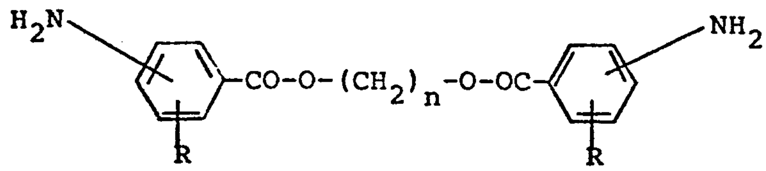

(1)

in welcher die Reste R gleich oder verschieden voneinander sein können und jedes für ein Wasserstoffatom, ein Chloratom, eine niedere Alkyl oder eine niedere Alkoxygruppe steht und n die Zahl 2, 3 oder 5 bis 8, vorzugsweise 3 oder 5, bedeutet, indem man von zwei Mol einer oder zwei verschiedener Nitrobenzoesäuren der allgemeinen Formel (2)

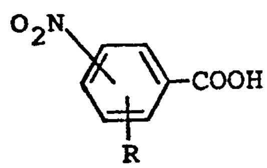

(2)

in welcher R die obengenannte Bedeutung hat, ausgeht, diese mit einem Mol eines oder mehrerer Alkandiole der allgemeinen Formel (3)

$$HO{-}(CH_2)_n{-}OH \qquad (3)$$

in welcher n die obengenannte Bedeutung hat, zu einer Verbindung der allgemeinen Formel (4)

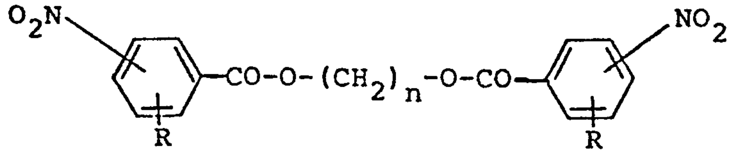

(4)

in welcher R und n die obengenannten Bedeutungen haben, umsetzt und anschließend die beiden Nitrogruppen reduziert.

Die oben verwendete Angabe « niedere » bedeutet, daß die erwähnten Verbindungen und Substituenten einen Alkylrest von 1 bis 4 C-Atomen enthalten. In den Formeln (1), (2) und (4) steht die Nitrogruppe bevorzugt in p-Stellung zur Carboxy- bzw. Carbonyloxygruppe. Aromatische Sulfonsäuren, die als Veresterungskatalysatoren erfindungsgemäß eingesetzt werden können, sind beispielsweise Benzol- und Naphthalinsulfonsäuren mit 1 bis 3 Sulfogruppen, die noch durch niederes Alkyl, wie Methyl und Äthyl, und/oder Halogen, wie Chlor, substituiert sein können, wie die Benzolsulfonsäure, die Toluolsulfonsäuren, die Xylolsulfonsäuren, die Chlorbenzolsulfonsäuren und die verschiedenen Naphthalinsulfonsäuren.

Das erfindungsgemäße Verfahren verläuft nicht nur in der Reduktionsstufe mit sehr hoher Ausbeute, sondern auch in der Veresterungsstufe praktisch quantitativ ; es entsteht kein Monoester. Eventuell bei der Veresterungsstufe in geringer Menge gebildete Nebenprodukte sowie der verwendete Veresterungskatalysator erscheinen nach der erfindungsgemäßen reduktiven Weiterverarbeitung in der Mutterlauge und werden auf diese Weise von dem ausgefällten Aminobenzoesäure-diester abgetrennt. Das erfindungsgemäße Verfahren zeichnet sich weiterhin durch eine sehr hohe Raum/Zeit-Ausbeute, eine niedrige Abwasserbelastung und eine sicherheitstechnisch unbedenkliche Verfahrensweise aus und ist in technisch einfachen Apparaturen durchführbar, so daß es insgesamt erhebliche Vorteile gegenüber den bekannten Verfahren besitzt.

Die nachstehenden Beispiele dienen zur Erläuterung der Erfindung. Die Teile sind Gewichtsteile, die Prozentangaben beziehen sich auf Gewichtsprozente, sofern nicht anders vermerkt.

### Beispiel 1

In eine auf 120 °C erwärmte Mischung aus 199,5 Teilen 1,3-Propandiol und 12,5 Teilen p-Toluolsulfonsäure werden unter Rühren innerhalb 30 Minuten 835 Teilen p-Nitrobenzoesäure eingetragen. Man erwärmt das Reaktionsgemisch auf 160 °C, wobei zügig Wasser abgespalten wird, das in einem Abscheider vom Reaktionsgemisch abgetrennt wird. Nach 2 bis 3 Stunden sind etwa 80 bis 83 Teile Wasser abdestilliert. Man legt nun Vakuum an und führt die Veresterung bei einem Druck von 135 mbar innerhalb von etwa 1 Stunde zu Ende. Die Gesamtmenge des danach abgeschiedenen Wassers beträgt 90 bis 91 Teile. Die erhaltene Schmelze des Bis-(p-nitrobenzoesäure)-1,3-propandiol-diesters, deren Erstarrungspunkt etwa bei 110 °C liegt, wird nun auf 120 °C abgekühlt ; man gibt zu ihr unter weiterem Rühren 4 000 Teile Methanol unter raschem Zulauf und erhält eine gut rührbare Suspension, zu der 25 Teile eines Nickelkatalysators, wie beispielsweise ein Katalysator aus 55 % Ni auf $SiO_2$, sowie 17 Teile Natriumdihydrogenphosphat-Monohydrat als Promotor zugesetzt werden. Man stellt die Suspension mit etwa 14 Teilen einer wäßrigen 33 %igen Natronlauge auf einen pH-Wert von 7, überführt sodann die neutrale Suspension in einen Autoklaven (sofern man die Veresterung nicht bereits in einer Apparatur vorgenommen hat, die auch als Autoklav verwendet werden kann) und hydriert mit Wasserstoff bei einem Druck von 45 bar und einer Reaktionstemperatur von 70 °C. Die Reaktionszeit beträgt etwa 1-3 Stunden. Anschließend entspannt man und drückt den Autoklaveninhalt in eine Vorlage, versetzt die Lösung unter Rühren mit 23 Teilen Aktivkohle und 23 Teilen Kieselgur und klärt bei 60 °C über ein Druckfilter. Anschließend gibt man dem Filtrat langsam 8 300 Teile Wasser zu, kühlt die erhaltene farblose Suspension unter Rühren auf 0 bis 10 °C ab, filtriert den Niederschlag ab, wäscht den Filterrückstand mit wenig Wasser methanolfrei und trocknet ihn unter reduziertem Druck bei etwa 50 °C bis zur Gewichtskonstanz.

Man erhält 730 Teile der farblosen Verbindung Bis-(p-amino-benzoesäure)-1,3-propandiol-diester der Formel

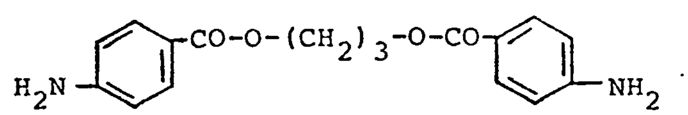

in einer theoretischen Ausbeute von 93 %. Sie hat einen Schmelzpunkt von 127 bis 128 °C.

### Beispiel 2

Man verfährt in gleicher Weise wie im Beispiel 1 beschrieben, arbeitet jedoch bei einer Hydriertemperatur von 75 °C und setzt als Alkandiol gleiche Mengen an 1,2-Propandiol ein. Man erhält 734 Teile des farblosen Bis-(p-aminobenzoesäure)-1,2-propandiol-diester der Formel

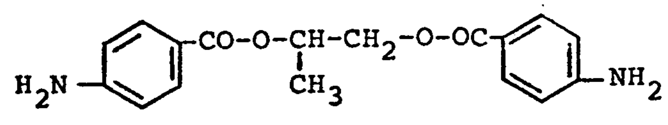

in einer Ausbeute von 93,5 % d. Th. mit einem Schmelzpunkt von 137 bis 138 °C.

Beispiele 3 bis 11

Verfährt man gemäß der erfindungsgemäßen Verfahrensweise zur Herstellung von Bis-aminobenzoesäure-alkandiol-diestern, beispielsweise analog der in Beispiel 1 beschriebenen Methode, und setzt die in den nachfolgenden Tabellenbeispielen angegebenen Nitrobenzoesäuren (gemäß Spalten I und II; Stellung der Substituenten bezogen auf die Carboxygruppe) und die in Spalte III angegebenen Alkandiole ein und verwendet die in Spalte IV angegebenen Veresterungskatalysatoren, die in Spalte V genannten Lösemittel und die in Spalte VI genannten Hydriertemperaturen, so erhält man die entsprechenden Aminobenzoesäure-diester (Stellung der Substituenten gemäß Spalten I und II, bezogen auf die Estergruppe) in hoher Reinheit mit den dort angegebenen Festpunkten und Ausbeuten.

(Siehe die Tabelle, Seite 7)

| Spalte | I | II | III | IV | V | VI | VII | VIII |
|---|---|---|---|---|---|---|---|---|
| Bsp. | Stellung d. $NH_2$-Gruppe bzw. $NO_2$-Gruppe | R | Alkandiol | Veresterungs-katalysator | Lösemittel | Hydrier-temp. in °C | Ausbeute (% d.Th.) | Fp. in °C |
| 3 | 2- | H | $HO-(CH_2)_6-OH$ | 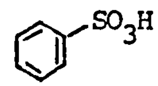 | Methanol | 45 | 91,3 | 74 - 75 |
| 4 | 4- | H | $HO-(CH_2)_5-OH$ | 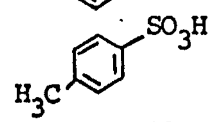 | Methanol | 45 | 92,5 | 135 - 136 |
| 5 | 4- | H | $HO-(CH_2)_2-OH$ | 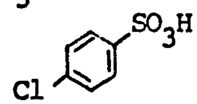 | Dimethyl-sulfoxid | 85 | 95,6 | 222 - 223 |
| 6 | 4- | 2-Cl | $HO-(CH_2)_2-OH$ | 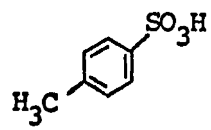 | Dimethyl-sulfoxid | 75 | 97,2 | 163 - 164 |
| 7 | 3- | $4-CH_3$ | $HO-(CH_2)_2-OH$ | dito | Dimethyl-sulfoxid | 85 | 96,4 | 148 - 149 |
| 8 | 4- | H | $HO-CH_2-\overset{CH_3}{\underset{CH_3}{C}}-CH_2-OH$ | dito | Äthanol | 50 | 90,5 | 155 - 156 |
| 9 | 4- | H | $HO-(CH_2)_3-OH$ | 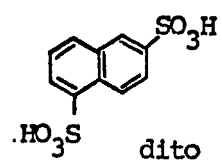 | i-Propanol | 45 | 93,0 | 127 - 128 |
| 10 | 4- | H | $HO-\overset{CH_3}{CH}-CH_2-OH$ | dito | Methanol | 75 | 94,0 | 137 - 138 |
| 11 | 4- | H | $HO(CH_2)_5OH$ | dito | Methanol | 45 | 91,5 | 135 - 136 |

**Ansprüche**

1. Verfahren zur Herstellung von Bis-(aminobenzoesäure)-alkandiol-diestern durch Veresterung von Alkandiolen, — ausgenommen solchen, bei denen die Hydroxygruppen in 1,4- oder 2,5-Stellung stehen —, mit Nitrobenzoesäuren und anschließende katalytische Reduktion der Bis-(nitrobenzoesäure)-alkandiol-diester-Verbindung in einem polaren, mit Wasser teilweise oder beliebig homogen mischbaren organischen Lösemittel, dadurch gekennzeichnet, daß man die Veresterung in Gegenwart einer aromatischen Sulfonsäure als Katalysator und in Abwesenheit eines Lösemittels und bei einer Temperatur zwischen 150 und 175 °C durchführt.

2. Verfahren nach Anspruch 1, dadurch gekennzeichnet, daß man die in dem polaren Lösemittel gelöste Aminobenzoesäure-diester-Verbindung aus dieser Lösung durch Zugabe von Wasser abscheidet.

3. Verfahren nach Anspruch 1, dadurch gekennzeichnet, daß man die Veresterung bei einer Temperatur zwischen 150 und 170 °C durchführt.

4. Verfahren nach Anspruch 1 oder 3, dadurch gekennzeichnet, daß man als Veresterungskatalysator eine Sulfosäure der Benzol- oder Naphthalinreihe verwendet.

5. Verfahren nach Anspruch 2, dadurch gekennzeichnet, daß die Zugabe von Wasser zur organischen Lösung so bemessen ist, daß der Anteil des polaren Lösemittels an der entstehenden wäßrig-organischen Lösung 25 bis 35 Gew.-% beträgt.

6. Verfahren nach Anspruch 1, dadurch gekennzeichnet, daß man nach der Veresterungsreaktion die Reaktionsschmelze nach dem Abkühlen auf eine Temperatur von 100 bis 125 °C mit einem polaren, mit Wasser teilweise oder beliebig homogen mischbaren organischen Lösemittel verdünnt und die Hydrierung als Eintopfverfahren weiterführt.

**Claims**

1. Process for the preparation of bis-(aminobenzoic acid)-alkanediol diesters by esterification of alkanediols — except those in which the hydroxy groups are in 1.4- or 2.5-positions — with nitrobenzoic acids and subsequent catalytic reduction of the bis-(nitrobenzoic acid)-alkanediol diester compound in a polar organic solvent, this solvent being partly or homogeneously miscible with water in all proportions, characterized by that the esterification is carried out in the presence of an aromatic sulfonic acid as a catalyst and in the absence of a solvent and at a temperature between 150 and 175 °C.

2. Process according to claim 1, characterized by that the aminobenzoic acid diester compound dissolved in the polar solvent is separated out of this solution by addition of water.

3. Process according to claim 1, characterized by that the esterification is carried out at a temperature between 150 and 170 °C.

4. Process according to claim 1 or 3, characterized by that a sulfonic acid of the benzene or naphthalene series is used as the esterification catalyst.

5. Process according to claim 2, characterized by that the addition of water to the organic solution is calculated in an amount such that the portion of the polar solvent in the aqueous-organic solution formed is 25 to 35 % by weight.

6. A process according to claim 1, characterized by diluting the reaction melt from the esterification reaction, after cooling it to a temperature of from 100 to 125 °C, with a polar solvent being partly or homogenously miscible with water in all proportions, and performing the hydrogenation as a single-pot-process.

**Revendications**

1. Procédé de préparation de bis-(aminobenzoates) d'alcane-diols par estérification d'alcane-diols autres que ceux dans lesquels les groupes hydroxy sont en position 1,4 ou 2,5, avec des acides nitrobenzoïques, puis réduction catalytique des bis-(nitro-benzoates) d'alcanes-diols dans un solvant organique polaire, partiellement miscible à l'eau ou miscible à l'eau en toute proportion, procédé caractérisé en ce qu'on effectue l'estérification en présence d'un acide sulfonique aromatique jouant le rôle de catalyseur et sans solvant, à une température comprise entre 150 et 175 °C.

2. Procédé selon la revendication 1 caractérisé en ce qu'on fait précipiter le diester aminobenzoïque dissous dans le solvant polaire en ajoutant de l'eau à cette solution.

3. Procédé selon la revendication 1 caractérisé en ce qu'on effectue l'estérification à une température comprise entre 150 et 170 °C.

4. Procédé selon l'une quelconque des revendications 1 à 3, caractérisé en ce qu'on utilise, comme catalyseur d'estérification, un acide sulfonique de la série benzénique ou de la série naphtalénique.

5. Procédé selon la revendication 2, caractérisé en ce qu'on règle la quantité d'eau que l'on ajoute à

la solution organique de telle façon que la teneur en solvant polaire de la solution organique-aqueuse formée soit de 25 à 35 % en poids.

6. Procédé selon la revendication 1, caractérisé en ce qu'après la réaction d'estérification on dilue la masse réactionnelle fondue, après l'avoir refroidie à une température de 100 à 125 °C, avec un solvant organique polaire, partiellement miscible à l'eau ou miscible à l'eau en toute proportion, puis on effectue l'hydrogénation, par un mode opératoire à un seul pot.